# EUROPEAN PATENT SPECIFICATION

(11) **EP 3 555 035 B1**
(45) Date of publication and mention of the grant of the patent: **04.08.2021**
(21) Application number: 17880374.8
(22) Date of filing: 14.12.2017
(51) Int. Cl.: C08F 4/20, C08F 112/08, C08F 112/12, C08F 116/18

(54) **INITIATOR SYSTEM FOR CATIONIC POLYMERIZATION OF OLEFINS**
INITIATORSYSTEM ZUR KATIONISCHEN POLYMERISIERUNG VON OLEFINEN
SYSTÈME INITIATEUR POUR LA POLYMÉRISATION CATIONIQUE D'OLÉFINES

(30) Priority: 16.12.2016 EP 16204669
(43) Date of publication of application: 23.10.2019
(73) Proprietor: ARLANXEO Canada Inc., Sarnia, ON N7T 7M2 (CA); The University of British Columbia, Vancouver, British Columbia V6T 1Z3 (CA)
(72) Inventor: ARSENAULT, Gilles, London, Ontario N6G0C2 (CA); KHATERA, Hazin, Vancouver, British Columbia V6T 1Z1 (CA); GATES, Derek, Vancouver, British Columbia V6T 1Z1 (CA)
(74) Representative: Kurz, Arnd
(86) International application number: PCT/CA2017/051517
(87) International publication number: WO 2018/107295

(56) References cited:
- CA-A1- 2 034 168
- CA-A1- 2 235 905
- DE-A1- 19 704 482
- US-A- 3 361 778
- US-A- 3 919 180
- US-A1- 2010 273 964
- US-A1- 2012 208 971
- TOMOYUKI TODA et al.: "Synthesis, Structure, and 1-Hexene Polymerization Catalytic Ability of Group 5 Metal Complexes Incorporating an [OSSO]-Type Ligand", ACS Catalysis, vol. 3, 2013, pages 1764-1767, XP055493115,
- KAZUSHI MASHIMA et al.: "Polymerization of Ethylene Catalyzed by the System Ta (n5-C5Me5 )(n4-diene)( CH 3)2/MAO: An Isoelectronic Analogue for a Group 4 Metallocene Catalyst", J. Am. Chem. Soc., vol. 115, 1993, pages 10990-10991, XP055493117,
- KAZUSHI MASHIMA et al.: "Living Polymerization of Ethylene Catalyzed by Diene Complexes of Niobium and Tantalum, M(n5-C5Me5)(n4-diene)X2 and M(n5-C5Me5 )(n4-diene)2(M=Nb and Ta), in the Presence ofMethylaluminoxane", Organometallics, vol. 14, 1995, pages 2633-2640, XP055600875,
- YIMIN SUN et al.: "Al-, Nb-, and Ta-Based Perfluoroaryloxide Anions as Cocatalysts for Metallocene-Mediated Ziegler-Natta Olefin Polymerization", Organometallics, vol. 19, 2000, pages 1625-1627, XP001126708,
- YAHYA AL-KHAFAJI et al.: "Tetraphenolate niobium and tantalum complexes for the ring opening polymerization of e-caprolactone", Dalton Trans., vol. 44, 2015, pages 12349-12356, XP055493124,

## Description

### Cross-reference to related Applications

This application claims priority from European Patent Application 16204669.2 filed December 16, 2016.

### Field

This application relates to a process for producing a polymer from one or more ethylenically unsaturated monomers. The application further relates to an initiator system for the process, and to compounds in the initiator system.

### Background

Various types of initiator systems for cationic polymerization of ethylenically unsaturated monomers are known in the art, including systems based on protonic or Bronsted-Lowry acids, Lewis acids (e.g. Friedel-Crafts catalysts), carbenium ion salts and ionizing radiation. Common protonic acids include phosphoric, sulfuric, fluoro-, and triflic acids, which tend to produce only low molecular weight polymers.

Lewis acids are the most common compounds used for initiation of cationic polymerization, and include, for example, SnCl₄, AlCl₃, BF₃ and TiCl₄. Although Lewis acids alone may be able to induce polymerization, the reaction occurs much faster with a co-initiator that acts as a suitable cation source (e.g. water, alcohols, HCI). However, such cationic polymerization reactions generally require very low temperature (about -100°C to about -90°C) to produce polymers of suitable molecular weight. Further, polymerization processes performed at such low temperatures are energy intensive; therefore, a process that can produce polymers with similar molecular weights at higher temperatures would significantly reduce the energy consumption and manufacturing cost of the process.

Recently, an initiator system for cationic polymerization has been developed based on a pentavalent phosphorus (V) complex with a dihydroxy compound (United States Patent Publication US 2012/0208971 published August 16, 2012). However, this initiator system produces low molecular weight products at higher temperatures, requiring lower temperatures to produce polymers of desirably high molecular weight. For example, the polymerization of α-methyl styrene at -50°C produces poly(a-methylstyrene) having Mₙ of less than about 7000 g/mol, Further, in order to produce polystyrene having Mₙ of greater than 100,000 g/mol, the polymerization must be done at temperatures lower than -80°C. The phosphorus complex can also be difficult to handle due to lack of stability.

There remains a need for initiator systems for cationic polymerization, which can produce suitably high molecular weight polymer at higher temperatures.

### Summary

A strong Brønsted-Lowry acid based on complexes of tantalum (V) ions or other isoelectronic metal ions (e.g. vanadium (V) or niobium (V) ions) provides an efficient initiator system for cationic polymerization of ethylenically unsaturated monomers at higher temperatures. High molecular weight polymers may be formed with the use of the present initiator system at higher temperatures.

In one aspect, there is provided a process for producing a polymer, the process comprising polymerizing one or more ethylenically unsaturated monomers under anhydrous conditions in presence of a Bronsted-Lowry acid polymerization initiator, the Brønsted-Lowry acid polymerization initiator having a structure of Formula (I): 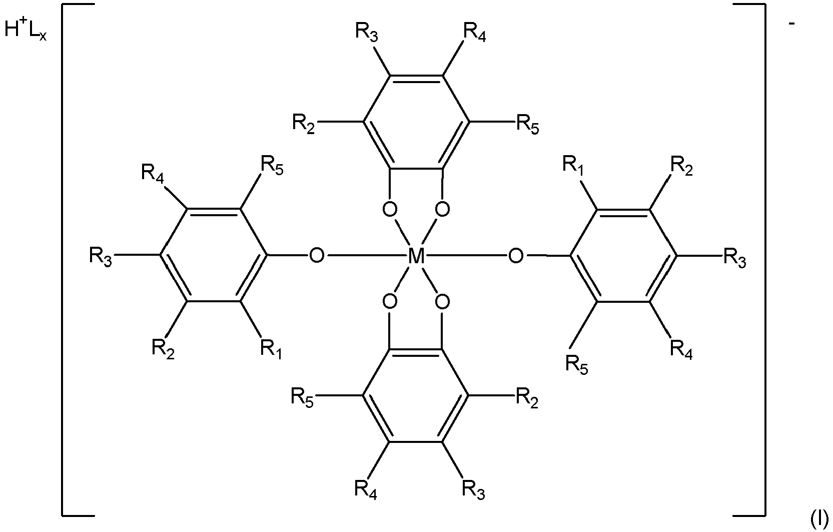 where:
M is tantalum (Ta), vanadium (V) or niobium (Nb);
each R₁ is independently H, OR₆, F, Cl, Br, I or alkyl, where R₆ is H or alkyl;
R₂, R₃, R₄ and R₅ are the same or different and are independently selected from H, F, Cl, Br, I,
alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present.

In another aspect, there is provided a Brønsted-Lowry acid initiator system for cationic polymerization of an ethylenically unsaturated monomer, the Brønsted-Lowry acid initiator system comprising an initiator having a structure of Formula (I) as defined above in an anhydrous polymerization medium.

In another aspect, there is provided a compound of Formula (I), where M, R₁, R₂, R₃, R₄, R₅, L and x are as defined above.

Further features will be described or will become apparent in the course of the following detailed description. It should be understood that each feature described herein may be utilized in any combination with any one or more of the other described features, and that each feature does not necessarily rely on the presence of another feature except where evident to one of skill in the art.

### Brief Description of the Drawings

For clearer understanding, preferred embodiments will now be described in detail by way of example, with reference to the accompanying drawings, in which:

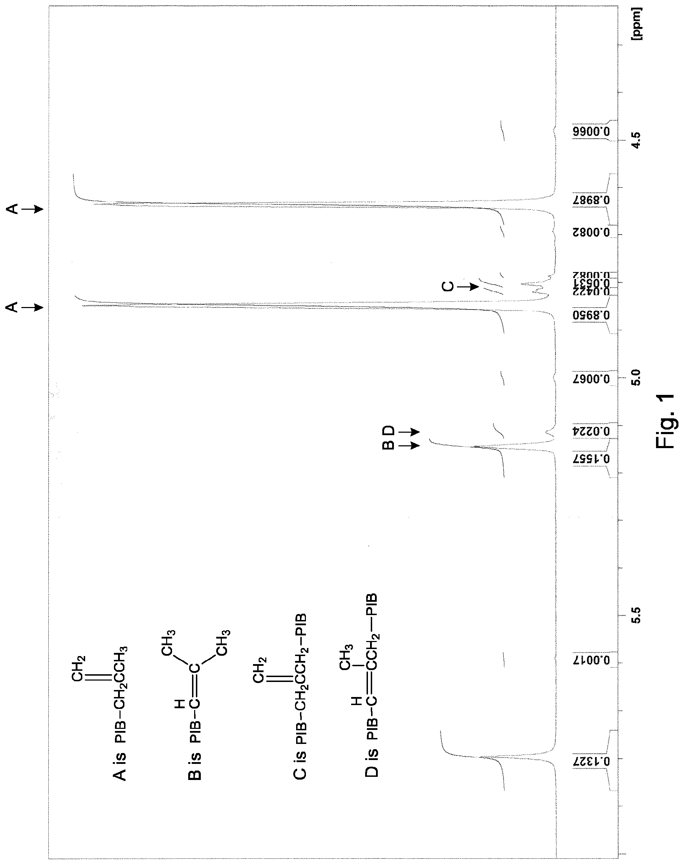

Fig. 1 is a ¹H NMR spectrum of polyisobutylene (PIB) produced using Initiator (VII).

### Detailed Description

The strong Brønsted-Lowry acid comprises a metal complex of organic ligands as described above for Formula (I). Alkyl is preferably C₁₋₆ alkyl, more preferably C₁₋₄ alkyl (e.g. methyl, ethyl, n-propyl, *i*-propyl, n-butyl, s-butyl, *t*-butyl), even more preferably methyl. Alkyl may be unsubstituted or substituted by one or more substituents. Substituents may be, for example, F, Cl, Br or aryl. Aryl is preferably C₁₋₁₈ aryl, more preferably C₁₋₁₀ aryl, even more preferably C₁₋₆ aryl, for example phenyl. Aryl may be unsubstituted or substituted by one or more substituents. Substituents may be, for example, F, Cl, Br or alkyl, where alkyl is as defined above.

M is preferably tantalum.

R₁ is preferably independently H, OH or CH₃. More preferably, each R₁ is the same and is H, OH or CH₃.

When two or more of R₂, R₃, R₄ and R₅ on a same benzene ring are taken together to form a bicyclic, tricyclic or tetracyclic moiety with the benzene ring, the moiety is preferably a fused ring system, for example a naphthyl moiety or an anthracyl moiety. R₂, R₃, R₄ and R₅ are preferably independently H, F, Cl, Br, I, alkyl or aryl, with the proviso that all of R₂, R₃, R₄ and R₅ on the same benzene ring are not H. More preferably, R₂, R₃, R₄ and R₅ are independently H, F, Cl or Br, with the proviso that all of R₂, R₃, R₄ and R₅ on the same benzene ring are not H. When R₂, R₃, R₄ and R₅ on the same benzene ring are both hydrogen and halogen (e.g. F, Cl, Br), the benzene ring may be mono-, di- or tri-halogenated, preferably di- or tri-halogenated. Preferably, R₂, R₃, R₄ and R₅ are all independently F, Cl or Br. More preferably, R₂, R₃, R₄ and R₅ are independently F or Cl. Even more preferably, R₂, R₃, R₄ and R₅ are the same and are F or Cl, which provides for tetra-fluorinated or tetra-chlorinated benzene rings.

In one embodiment, M is Ta; each R₁ is H, F, Cl, OH or CH₃; R₂, R₃, R₄ and R₅ are F or Cl; L is Et₂O and x is 2.

In another embodiment, M is Ta; each R₁ is H, OH, OCH₃ or CH₃; R₂, R₃, R₄ and R₅ are F or Cl; L is Et₂O and x is 2.

The Brønsted-Lowry acid polymerization initiator is particularly useful for initiating the polymerization or copolymerization of ethylenically unsaturated monomers. Ethylenically unsaturated monomers are compounds having at least one olefin bond therein. The monomers preferably comprise from 2 to 20 carbon atoms. Some examples of ethylenically unsaturated monomers include alkyl vinyl compounds (e.g. alkyl vinyl ethers and the like), aryl vinyl compounds (e.g. styrene, *α*-methylstyrene, p-methylstyrene, p-methoxystyrene, 1-vinylnaphthalene, 2-vinylnaphthalene, 4-vinyltoluene and the like) and isoprene. Of particular note are n-butyl vinyl ether, styrene, α-methylstyrene and isoprene.

Polymers formed from the polymerization of the monomers may be homopolymers, copolymers, terpolymers or other forms of polymers. The polymers may be linear, branched or star branched. Mixtures of two or more monomers may be polymerized into copolymers or terpolymers. Some examples of polymers include polystyrene, poly(a-methylstyrene), poly(N-vinylcarbazole), polyterpenes, polyisoprenes, polyisobutylenes and the like. Of particular note are copolymers of isobutylene and isoprene (e.g. butyl rubber), polyisobutylene, polyisoprene polystyrenes (e.g. polystyrene and poly(a-methylstyrene) and poly(n-butyl vinyl ether).

Polymers produced in the polymerization of ethylenically unsaturated monomers may have number average molecular weights (Mₙ) of at least about 2,000 g/mol, or at least about 5,000 g/mol, or at least about 10,000 g/mol, or at least about 20,000 g/mol, or at least about 30,000 g/mol, or at least about 50,000 g/mol, or at least about 100,000 g/mol, depending on the monomer or momomers undergoing polymerization, the relative amounts of monomer and initiator, the temperature at which the polymerization is conducted and other process conditions. The polymer may have number average molecular weights (Mₙ) up to about 1,000,000 g/mol, or up to about 500,000 g/mol, or up to about 250,000 g/mol.

The initiator is a cationic initiator because the initiator is a Brønsted-Lowry acid, thereby further comprising a hydrogen ion (H⁺) as counterion to an anionic metal complex. The hydrogen ion may be associated as a "naked" ion with the metal complex (i.e. x = 0). To stabilize the hydrogen ion, the initiator may further comprise a stabilizing molecule (L) for the hydrogen ion. The stabilizing molecule is a molecule that is able to stabilize the hydrogen ion without making the hydrogen ion unavailable for catalyzing the polymerization. The value of x may be an integer or a fractional number depending on whether H⁺ ions associated with neighboring complexes in a bulk material of the polymerization initiator share a molecule, L. When a molecule L is shared between neighboring H⁺ ions, the value of x may be fractional. The value of x is preferably 0.5, 1, 1.5, 2, 2.5 or 3. In one embodiment, there are two stabilizing molecules for each H⁺ ion (i.e. x = 2). The stabilizing molecule may be a molecule that can form hydrogen bonds with the hydrogen ion. The stabilizing molecule may therefore contain one or more atoms that have lone pairs of electrons, for example O or N atoms. Sterically-hindered stabilizing molecules having one or more lone pairs of electrons are particularly useful as they sufficiently stabilize the hydrogen ion while permitting the hydrogen ion to initiate carbocationic polymerization. Some examples of stabilizing molecules include ethers and the like. Aprotic stabilizing molecules are preferred. Alkyl and cycloalkyl ethers are particularly preferred. Some examples of suitable stabilizing molecules are tetrahydrofuran, tetrahydropyran, dioxane, dimethyl ether, diethyl ether, bis(2-chloroethyl) ether, dipropyl ether, diisopropyl ether, methyl ethyl ether, methyl n-propyl ether, methyl isopropyl ether, bis(2-chloroisopropyl) ether, methyl tert-butyl ether, ethyl tert-butyl ether, diisobutyl ether, dihexyl ether, 2,5-dimethyltetrahydrofuran, 2-chloro ethyl ether, 2-methyltetrahydrofuran, cyclopentyl methyl ether, diethylene glycol dimethyl ether (diglyme), tetraethylene glycol dimethyl ether, diphenyl ether, 2,6-di-tert-butyl pyridine and the like. In one embodiment, the stabilizing molecule is diethyl ether. Where the stabilizing molecule is a solvent, the stabilizing molecule may form a solvate with the hydrogen ion.

The compound of Formula (I) may be synthesized by contacting a metal ion precursor compound in a reaction mixture with an organic α-,β-dihydroxy ligand compound of Formula (IIa), or by contacting the metal ion precursor compound with an organic α-,β-dihydroxy ligand compound of Formula (IIa) and an organic monohydroxy ligand compound of Formula (IIb): 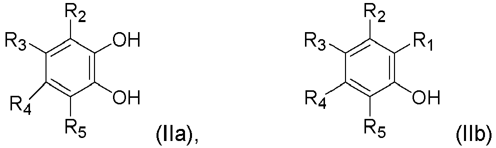 where R₁ and R₂ are as defined above, with the proviso that R₁ in the compound of Formula (IIb) is not OH. Mixtures of different organic ligand compounds may be used.

The metal ion precursor compound and organic ligand compounds may be present in the reaction mixture in amounts to provide a molar ratio that results in the metal complex having sufficient ligands to provide a negative charge to the metal complex. To provide metal complexes where R₁ is OH, about 4 molar equivalents of the organic α-,β-dihydroxy ligand compound of Formula (IIa) is suitable to result in the metal complex having four ligands, two bidentate ligands and two monodentate ligands, thereby providing the metal complex with a -1 charge. To provide metal complexes where R₁ is other than OH, about 2 molar equivalents of the organic α-,β-dihydroxy ligand compound of Formula (IIa) and 2 molar equivalents of the organic monohydroxy ligand compound of Formula (IIb) are suitable to result in the metal complex having four ligands, two bidentate ligands and two monodentate ligands, thereby providing the metal complex with a -1 charge. Where both the compounds of Formula (IIa) and (IIb) are reacted with the metal ion precursor compound, the compound of Formula (IIa) is preferably reacted first with the metal ion precursor compound to produce an intermediate metal complex, followed by further reaction with the compound of Formula (IIb) to produce the compound of Formula (I).

The metal ion precursor compound may be a compound of a metal ion with leaving groups as ligands. Suitable leaving groups include, for example, halogen (CI, Br), CO, CN and the like. The metal ion precursor compound and organic ligand compounds are preferably dry and high purity. Contacting the metal ion precursor compound with the organic ligand compounds may be performed in the presence or absence of a solvent, preferably in the presence of a solvent. The solvent may comprise an aprotic organic solvent, preferably a non-coordinating solvent. Some examples of suitable solvents include alkyl halides (e.g. dichloromethane), aromatic hydrocarbons (e.g. toluene) and acetonitrile. A stabilizing molecule for hydrogen ions may be included in the reaction mixture, preferably after the metal complex is formed, to solvate the hydrogen ion. The reaction is preferably conducted under anhydrous conditions. The reaction may be conducted at elevated temperature, for example by refluxing the solvent. The reaction may be conducted for an amount of time sufficient to maximize the yield of the initiator, for example for a time up to about 3 hours. The reaction is preferably conducted by slowly adding the ligand compound to a reaction mixture containing the metal ion precursor compound, although other addition schemes may be used. The initiator may be recovered from the reaction mixture by standard techniques, for example filtration, washing, recrystallization and drying.

The initiator is preferably used in amount to provide a monomer to initiator mole ratio ([M]:[I]) of at least about 20:1. A higher [M]:[I] may be preferred in some embodiments to produce high yields of high molecular weight polymer. In some embodiments, the [M]:[I] may be at least about 100:1. In some embodiments, the [M]:[I] may be in a range of about 100:1 to about 1000:1, or about 200:1 to about 800:1, or about 300:1 to about 500:1.

The polymerization is generally conducted in a polymerization medium. The polymerization medium may be provided, for example, by a solvent or diluent. Solvents or diluents for the polymerization may include, for example a halogenated organic liquid, a non-halogenated organic liquid or mixtures thereof. Halogenated organic liquids include, for example, chlorinated or fluorinated organic compounds. Chlorinated organic compounds include, for example C1-C4 alkyl chlorides (e.g. dichloromethane (DCM) and methyl chloride (MeCl)). DCM is generally useful as a solvent for solution polymerization, while MeCI is generally useful as a diluent for slurry polymerization. Fluorinated organic compounds include, for example, hydrofluorocarbons (HFC) such as 1,1,1,2-tetrafluoroethane and the like, and hydrofluorinated olefins (HFO) such as 2,3,3,3-tetrafluoro-1-propene and the like. Fluorinated organic compounds are generally useful as diluents for slurry polymerization. Non-halogenated organic liquids include, for example, aliphatic hydrocarbons (e.g. cyclohexane, cyclopentane, 2,2-dimethylbutane, 2,3-dimethylbutane, 2-methylpentane, 3-methylpentane, n-hexane, methylcyclopentane and 2,2-dimethylpentane). Halogenated organic solvents, in particular C1-C4 alkyl chlorides are preferred. Dichloromethane (CH₂Cl₂) or methyl chloride (MeCI) are particularly preferred.

The solvent or diluent is preferably present in the polymerization medium in an amount of about 10-80 vol%, based on volume of the polymerization medium. In preferred embodiments, the medium may comprise a diluent in an amount of about 55-80 vol%, or a solvent in an amount of about 10-50 vol%.

The polymerization is conducted under anhydrous conditions. Preferably, water is present in an amount less than about 1 ppm, more preferably less than about 0.5 ppm, yet more preferably less than about 0.1 ppm. It is preferable to eliminate water from the polymerization medium altogether. Reducing or eliminating moisture in the polymerization medium helps to produce polymers having higher molecular weights at higher yields.

It is an advantage of the present initiator system that the polymerization may be conducted at a higher temperature than with other Brønsted-Lowry acid or Lewis acid initiator systems, while being able to produce suitably high molecular weight polymers at good yield. The temperature at which the polymerization is conducted may be -90°C or higher, or -85°C or higher, or -80°C or higher, or -70°C or higher, or -60°C or higher, or -50°C, or -40°C or higher. The temperature may be as high as 30°C or lower, or 20°C or lower, or 10°C or lower, or 0°C or lower, or -10°C or lower, or -15°C or lower, or -20°C or lower, or -25°C or lower, -30°C or lower, or -35°C or lower.

### EXAMPLES:

### General Materials and Procedures:

All experiments were performed using standard Schlenk or glove box techniques under nitrogen atmosphere.

Dichloromethane (CH₂Cl₂) and diethyl ether (Et₂O) were deoxygenated with nitrogen and dried by passing through a column containing activated alumina. Tetrahydrofuran (THF) (Fisher Scientific) was dried and distilled over benzophenone ketyl prior to use. CH₂Cl₂ (Sigma Aldrich), Et₂O (Fisher Scientific), styrene (Sigma Aldrich) and n-butyl vinyl ether (Sigma Aldrich) were dried over calcium hydride, distilled and freeze-pump-thaw (x3) degassed prior to use. CH₂Cl₂, Et₂O and methyl tert-butyl ether were stored over molecular sieves prior to use.

Tantalum pentachloride (Aldrich) and niobium pentachloride (Aldrich) were used without further purification. Tetrachlorocatechol was prepared following the procedure described in Lübbecke H., Boldt P. Tetrahedron 1978, 34, 1577-1579, the contents of which is herein incorporated by reference, and then azeotropically distilled and recrystallized from hot toluene prior to use. Tetrafluorocatechol was prepared following a literature procedure described in Barthel J, Buestrich R, Carl E, Gores HJ. J. Electrochem. Soc. 1996, 143, 3572-3575, the contents of which is herein incorporated by reference. Hexafluoro-2,3-bis(trifluoromethyl)-2,3-butanediol (Matrix Scientific) and 3-fluorocatechol (Sigma Aldrich) were used without further purification.
¹H and ¹³C{¹H} NMR spectra were recorded on Bruker Avance 300 or 400 MHz spectrometers at room temperature unless noted. ¹H NMR and ¹³C{¹H} NMR spectra were referenced to deuterated solvents.

Molecular weight of polymers was determined by triple detection gel permeation chromatography (GPC-LLS) utilizing an Agilent 1260 Series standard auto sampler, an Agilent 1260 series isocratic pump, Phenomenex Phenogel™ 5 µm narrowbore columns (4.6 x 300 mm) 10⁴ Å (5000-500,000), 500 Å (1,000-15,000), and 10³ Å (1,000-75,000), a Wyatt Optilab™ rEx differential refractometer (λ = 658 nm, 25 °C), as well as a Wyatt tristar miniDAWN (laser light scattering detector (λ = 690 nm)) and a Wyatt ViscoStar viscometer. Samples were dissolved in THF (ca. 2 mg mL⁻¹) and a flow rate of 0.5 mL min⁻¹ was applied. The differential refractive index (dn/dc) of poly(n-butyl vinyl ether) *(dn*/*dc =* 0.068 mL g⁻¹) in THF was calculated by using Wyatt ASTRA software 6.1. The differential refractive index (*dn*/*dc*) of poly(styrene) (*dn*/*dc* = 0.185 mL g⁻¹) and of poly(α-methylstyrene) (*dn*/*dc* = 0.204 mL g⁻¹) has been reported in McManus NT, Penlidis A. J. Appl. Polym. Sci. 1998, 70, 1253-1254. The differential refractive index (*dn*/*dc*) of poly(isoprene) (*dn*/*dc* = 0.129 mL g⁻¹) (Jackson C, Chen YJ, Mays JW. J. Appl. Polym. Sci. 1996, 61, 865) has been reported.

### Initiator (III):

### Synthesis of H(Et₂O)₂[Ta(1,2-O₂C₆Cl₄)₂(1,2-O₂H₁C₆Cl₄)₂] (III)

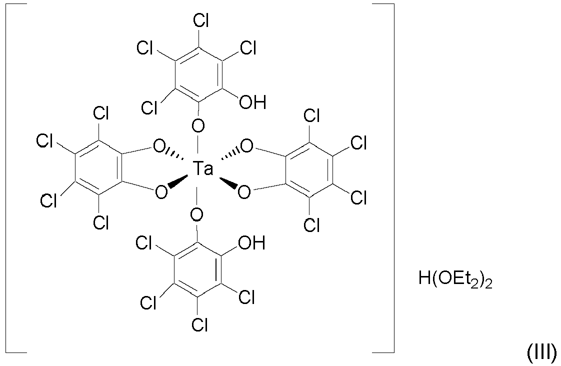

TaCl₅ (0.53 g, 1.5 mmol) was stirred in anhydrous CH₂Cl₂ (10 mL) and the white suspension was slowly heated to reflux. In another Schlenk flask, about 4 equivalents of tetrachlorocatechol (1.48 g, 5.9 mmol) was prepared in warm anhydrous CH₂Cl₂ (14 mL) and the bright orange-red solution was added via cannula to the refluxing TaCl₅ solution at 90°C to afford a dark green reaction mixture. After 10 minutes a colorless precipitate was obtained. The reaction mixture was refluxed for 80 minutes and cooled to ambient temperature. Upon addition of Et₂O (25 mL), a green clear solution formed. The solution was cooled in an ice bath to afford an off-white precipitate within 15 minutes. The solid was collected by filtration, washed with CH₂Cl₂ (5 mL) and dried *in vacuo.* Yield = 1.13 g, 0.9 mmol, 66 %. A concentrated solution of the crude product in CH₂Cl₂ afforded colorless crystals of H(Et₂O)₂[Ta(1,2-O₂C₆Cl₄)₂(1,2-O₂H₁C₆Cl₄)₂] **(III)** (-30°C, ca. 3 d).
¹H NMR (400 MHz, CD₂Cl₂, 25°C): δ = 9.37 (br s, 3H, *H*O & *H*(Et₂O)₂), 4.00 (q, 8H, J = 7 Hz, C*H*₂CH₃), 1.40 ppm (t, 12H, J = 7 Hz, CH₂C*H*₃).
¹H NMR (400 MHz, CD₂Cl₂, -85°C): δ = 16.73 (s, 1H, *H*(Et₂O)₂), 9.40 (s, 2H, HO), 4.03 (q, 8H, J = 7 Hz, C*H*₂CH₃), 1.38 ppm (t, 6H, J = 7 Hz, CH₂C*H*₃).
¹³C{¹H} NMR (75 MHz, CD₂Cl₂, -85°C): δ = 150.0 (s), 145.3 (s), 144.3 (s), 139.8 (s), 125.0 (s), 121.6 (s), 121.1 (s), 118.6 (s), 116.7 (s), 70.3 (s), 13.3 ppm (s).

Elemental analysis (%) found: C, 28.30; H, 1.80. Calcd. for C₃₂H₂₃Cl₁₆O₁₀Ta·CH₂Cl₂: C, 28.31; H,1.80.

### Polymerization of monomers using initiator (III)

Polymerization of monomers with initiator (III) was performed by the following general procedure.

Initiator and monomer are initially stored at -30°C inside a freezer in a glovebox under a positive atmosphere of dry N₂ gas. The initiator (0.010 g, 0.010 mmol) is transferred to a 25 mL Schlenk flask, which is sealed with a rubber septum and then brought outside the glovebox maintaining isolation from the outside atmosphere to be connected to a dry N₂ gas line. The initiator in the flask is cooled to -78°C with an acetone/ dry ice bath. Anhydrous, degassed CH₂Cl₂ (2.0 mL) stored over activated molecular sieves is added via syringe to the initiator under a flow of dry N₂ gas and stirred to guarantee a homogenous solution at -78°C. The mixture is kept at -78°C for 10 minutes, or warmed or cooled to a different desired temperature and held at that temperature for 10 minutes, before addition of the monomer.

Freshly prepared and degassed monomer in an amount to achieve a desired monomer to initiator ratio ([M]:[I]) is collected in a 1 ml single-use plastic syringe inside the glovebox. The monomer is then injected rapidly through the rubber septum on the Schlenk flask into the initiator solution at the desired temperature under a constant flow of dry N₂ gas, and the reaction mixture is continuously stirred for 15 minutes while polymerization occurs. After the 15 minutes, the reaction is quenched with 0.2 mL of a solution of NH₄OH in MeOH (10 vol%), the Schlenk flask is removed from the cooling bath and all volatiles are removed *in vacuo.* The crude product is dissolved in 2 mL CH₂Cl₂ and added one drop at a time via syringe to vigorously stirred MeOH (40 mL) to precipitate an oily residue. The polymer is collected by centrifugation and dried *in vacuo.* Absolute molecular weight (Mₙ) is determined using triple-detection GPC.

### Effect of temperature on n-butyl vinyl ether polymerization

Table 1 shows data for the polymerization of *n*-butyl vinyl ether using initiator (III) at different temperatures. The data for each example represents the average of at least three separate polymerization reactions. Mₙ calc. = 40,000 g/mol. Table 1 shows that significant yield of poly(*n*-butyl vinyl ether) having a reasonably high molecular weight (Mₙ) can be achieved at temperatures well above -90°C.

**Table 1**

| Ex. | T (°C) | [M]:[I] | Yield (%) | Mₙ (g/mol) | PDI |
|---|---|---|---|---|---|
| 1 | 18 | 400 | 38 | 19,800 | 1.69 |
| 2 | 0 | 400 | 40 | 17,000 | 1.21 |
| 3 | -15 | 400 | 65 | 25,300 | 1.89 |
| 4 | -38 | 400 | 62 | 30,600 | 1.96 |
| 5 | -50 | 400 | 68 | 29,400 | 2.07 |
| 6 | -78 | 400 | 71 | 32,200 | 1.58 |
| 7 | -84 | 400 | 54 | 39,100 | 1.12 |

### Effect of monomer to initiator ratio ([M]:[I]) on n-butyl vinyl ether polymerization

Table 2 shows data for the polymerization of *n*-butyl vinyl ether using initiator (III) at different monomer to initiator ratios. The data for each example represents the average of at least three separate polymerization reactions. Table 2 shows that Mₙ of poly(n-butyl vinyl ether) can be increased by increasing [M]:[I] while keeping yield relatively constant.

**Table 2**

| Ex. | T(°C) | [M]:[I] | Yield (%) | Mₙ (g/mol) | PDI |
|---|---|---|---|---|---|
| 7 | -84 | 200 | 36 | 43,800 | 1.09 |
| 8 | -84 | 400 | 54 | 39,100 | 1.12 |
| 9 | -84 | 800 | 52 | 73,000 | 1.13 |

### Effect of temperature on styrene polymerization

Table 3 shows data for the polymerization of styrene using initiator (III) at different temperatures. The data for each example represents the average of at least three separate polymerization reactions. Mₙ calc. = 40,000 g/mol. Table 3 shows that an excellent balance between high yield and high molecular weight of polystyrene can be achieved at temperatures much higher than -90°C.

**Table 3**

| Ex. | T (°C) | [M]:[I] | Yield (%) | Mₙ (g/mol) | PDI |
|---|---|---|---|---|---|
| 10 | 19 | 400 | 67 | 6,100 | 2.16 |
| 11 | 0 | 400 | 82 | 9,500 | 2.94 |
| 12 | -15 | 400 | 85 | 12,800 | 3.51 |
| 13 | -38 | 400 | 72 | 131,500 | 1.34 |
| 14 | -50 | 400 | 78 | 147,100 | 1.43 |
| 15 | -78 | 400 | 7 | 205,600 | 1.29 |

### Effect of monomer to initiator ratio ([M]:[I]) on styrene polymerization

Table 4 shows data for the polymerization of *styrene* using initiator (III) at different monomer to initiator ratios. The data for each example represents the average of at least three separate polymerization reactions. Table 4 shows that Mₙ of styrene can be increased by increasing [M]:[I] while keeping yield relatively constant.

**Table 4**

| Ex. | T (°C) | [M]:[I] | Yield (%) | Mₙ (g/mol) | PDI |
|---|---|---|---|---|---|
| 16 | -50 | 200 | 72 | 147,100 | 1.37 |
| 17 | -50 | 492 | 78 | 147,100 | 1.43 |
| 18 | -50 | 800 | 72 | 106,000 | 1.76 |

### Effect of the presence of water on styrene polymerization

Table 5 shows data for the polymerization of styrene using initiator (III) at different temperatures in the presence of different amounts of trace moisture. Distilled water in the indicated amounts was added to the anhydrous CH₂Cl₂ prior to polymerization. Table 5 shows that the presence of trace moisture can drastically reduce yield and molecular weight of the polystyrene.

**Table 5**

| Ex. | T (°C) | H₂O (ppm) | [M]:[I] | Yield (%) | Mₙ (g/mol) | PDI |
|---|---|---|---|---|---|---|
| 19 | -50 | 0 | 400 | 78 | 145,000 | 1.43 |
| 20 | -50 | 1 | 400 | 1.6 | 54,800 | 1.42 |
| 21 | -50 | 5 | 400 | 0.9 | 67,700 | 1.58 |
| 22 | -50 | 10 | 400 | 0.8 | 59,900 | 1.62 |
| 23 | -50 | 100 | 400 | 0.6 | 17,110 | 1.84 |
| 24 | -78 | 0 | 400 | 5 | 205,600 | 1.29 |
| 25 | -78 | 1 | 400 | 1.6 | 121,800 | 1.39 |
| 26 | -78 | 5 | 400 | 1.3 | 102,800 | 1.44 |
| 27 | -78 | 10 | 400 | 1.0 | 68,200 | 1.78 |
| 28 | -78 | 100 | 400 | --- | --- | --- |

### Effect of temperature on α-methyl styrene polymerization

Table 6 shows data for the polymerization of α-methyl styrene using initiator (III) at different temperatures. The data for each example represents the average of at least three separate polymerization reactions. Mₙ calc. = 40,000 g/mol. Table 6 shows that good balance of high yield and high molecular weight for poly(a-methylstyrene) can be achieved at temperatures much higher than -90°C.

**Table 6**

| Ex. | T(°C) | [M]:[I] | Yield (%) | Mₙ (g/mol) | PDI |
|---|---|---|---|---|---|
| 29 | 19 | 400 | 1 | n.d. | n.d. |
| 30 | 0 | 400 | 3 | 5,100 | 1.63 |
| 31 | -15 | 400 | 45 | 4,800 | 2.37 |
| 32 | -38 | 400 | 63 | 10,100 | 1.87 |
| 33 | -50 | 400 | 82 | 66,400 | 1.81 |
| 34 | -78 | 400 | 56 | 241,000 | 1.32 |

| | | | | | |
|---|---|---|---|---|---|
| n.d. = not determined | | | | | |

### Effect of temperature on isoprene polymerization

Table 7 shows data for the polymerization of isoprene using initiator (III) at different temperatures. The data for each example represents the average of at least three separate polymerization reactions. Mₙ calc. = 27,200 g/mol. Table 7 shows that a high yield and good molecular weight for poly(isoprene) can be achieved at temperatures much higher than -90°C.

**Table 7**

| Ex. | T(°C) | [M]:[I] | Yield (%) | Mₙ (g/mol) | PDI |
|---|---|---|---|---|---|
| 35 | 18 | 400 | 70 | 3,000 | 2.13 |
| 36 | 0 | 400 | 52 | 3,200 | 4.84 |
| 37 | -15 | 400 | 65 | 2,100 | 5.01 |
| 38 | -38 | 400 | 55 | 2,900 | 1.52 |
| 39 | -50 | 400 | 40 | 2,600 | 1.73 |
| 40 | -78 | 400 | 5 | n.d. | n.d. |

| | | | | | |
|---|---|---|---|---|---|
| n.d. = not determined | | | | | |

### Initiator (IV):

### Synthesis of H(Et₂O)₂[Ta(1,2-O₂C₆F₄)₂(1,2-O₂H₁C₆F₄)₂] (IV)

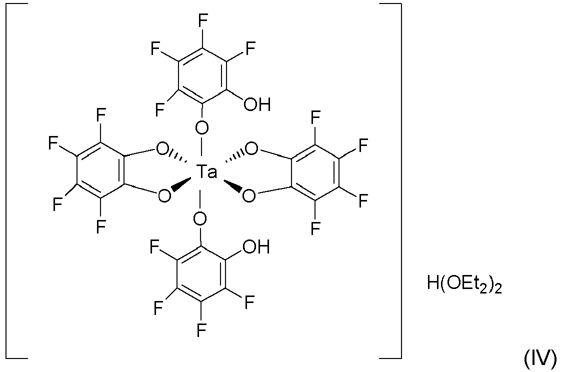

The reaction of 4 equivalents of tetrafluorocatechol with TaCl₅ in a manner as described for the synthesis of the chlorinated analog (III) affords the corresponding H(Et₂O)₂[Ta(1,2-O₂C₆F₄)₂(1,2-O₂H₁C₆F₄)₂] (IV).

Thus, TaCl₅ (0.09 g, 0.25 mmol) was stirred in anhydrous CH₂Cl₂ (3 mL) and the white suspension was slowly heated to reflux. In another Schlenk flask, tetrafluorocatechol (0.19 g, 1.05 mmol) was prepared in a warm anhydrous CH₂Cl₂ (6 mL) solution and the clear colorless solution was added via cannula to the refluxing TaCl₅ solution at 90°C to afford a light yellow-brown clear reaction mixture. After 25 min, a colorless precipitate was obtained. The reaction mixture was refluxed for 120 min and cooled to ambient temperature. Upon addition of Et₂O (20 mL), a light brown clear solution formed. The solution was cooled in an ice bath to afford a small amount of a faint brown colored precipitate within 30 min. The reaction mixture was pumped down to dryness, washed with cold Et₂O (2 mL) and dried in *vacuo* to give a faint brown colored oily residue. Yield = (0.23 g, 0.20 mmol, 77% based on TaCl₅).
¹H NMR (400 MHz, CD₂Cl₂, 25 °C): δ = 11.3 (br, *H*(Et₂O)₂, 4.00 (q, ³*J*_{HH} = 6.1 Hz, 8H, C*H*₂CH₃), 1.31 ppm (t, ³*J*_{HH} = 6.7 Hz, 12H, CH₂C*H*₃).
¹H NMR (400 MHz, CD₂Cl₂, -85°C): δ = 16.82 (s, 1H, *H*(Et₂O)₂), 9.72 (s, 1H, O*H*), 4.11 (q, ³*J*_{HH} = 6.7 Hz, 8H, C*H*₂CH₃), 1.44 (t, ³*J*_{HH} = 7.0 Hz, 12H, CH₂C*H*₃).
¹³C{¹H} NMR (75 MHz, CD₂Cl₂, -85 °C): δ = 138.4 (s, Ar-C), 136.1 (s, Ar-C), 133.6 (s, Ar-C), 131.9 (s, Ar-C), 128.1 (s, Ar-C), 128.9 (d, ²*J*_{TaC}= 12.4 Hz, Ar-C), 128.1 (s, Ar-C), 126.8 (s, Ar-C), 113.1 (s, Ar-C), 70.9 (s, OCH₂CH₃), 13.8 (s, OCH₂CH₃).
¹⁹F{¹H}NMR (282 MHz, CD₂Cl₂, -85 °C): δ = 160.9-161.1 (dd, ²*J*_{CF} = 9.5 Hz, O₂C₆F₄), 165.3 (br, O₂C₆H₁F₄), 167.7-167.8 (dd, ²*J*_{CF} = 9.5 Hz, O₂C₆F₄), 170.7 (br, O₂C₆H₁F₄) ppm.

### Polymerization of monomers using initiator (IV)

Polymerization of monomers with initiator (IV) was performed by following the general procedure described above for initiator (III). Table 8 shows data for the polymerization of *n*-butyl vinyl ether, styrene and α-methyl styrene using initiator (IV). Table 8 shows that good balance of high yield and high molecular weight for poly(n-butyl vinyl ether), poly(styrene) and poly(a-methylstyrene) can be achieved at temperatures much higher than -90°C.

**Table 8**

| Ex. | Monomer | T (°C) | [M]:[I] | Yield (%) | Mₙ (g/mol) | PDI |
|---|---|---|---|---|---|---|
| 41 | *n*-butyl vinyl ether | -78 | 400 | 88 | 109,000 | 1.29 |
| 42 | styrene | -50 | 400 | 15 | 26,200 | 1.52 |
| 43 | α-metyl styrene | -50 | 400 | 53 | 207,100 | 1.31 |
| 44 | α-metyl styrene | -78 | 400 | 10 | 184,100 | 1.46 |

### Initiator (V):

### Synthesis of H(THF)₂[Ta(1,2-O₂C₆Cl₄)₂(1,2-O₂H₁C₆Cl₄)₂] (V)

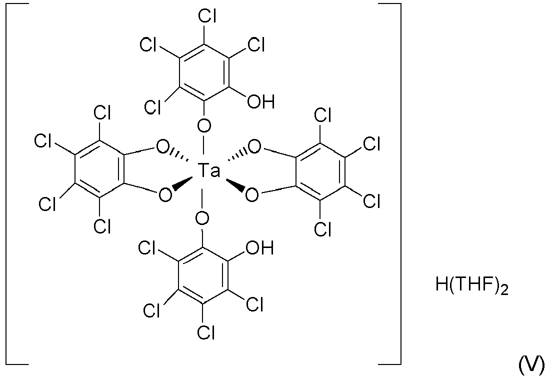

The synthesis of initiator (III) described above may be adapted to replace diethyl ether with tetrahydrofuran (THF) as the coordinating ligand for the proton to afford H(THF)₂[Ta(1,2-O₂C₆Cl₄)₂(1,2-O₂H₁C₆Cl₄)₂] (V).

Thus, TaCl₅ (0.43 g, 1.20 mmol) was stirred in anhydrous CH₂Cl₂ (6 mL) and the white suspension was slowly heated to reflux under N₂ atmosphere. In another Schlenk flask, tetrachlorocatechol (1.20 g, 4.85 mmol) was prepared in warm anhydrous CH₂Cl₂ (6 mL) and the bright orange-red solution was added via cannula to the refluxing TaCl₅ solution at 90°C to afford a dark green reaction mixture. After 10 min, a colorless precipitate was obtained. The reaction mixture was refluxed for 80 min and cooled to ambient temperature. Upon addition of THF (2.4 mL), a green clear solution formed. The solution was cooled in an ice bath to afford a light green precipitate within 30 min. The solid was collected by filtration, washed with CH₂Cl₂ (2 mL) and dried in *vacuo.* Yield = (0.86 g, 0.66 mmol, 55% based on TaCl₅).
¹H NMR (400 MHz, CD₂Cl₂, 25 °C): δ = 8.18 (br, OH), 3.91 (br, 8H, OC*H*₂CH₂), 1.95 ppm (br, 8H, OCH₂C*H*₂).
¹H NMR (400 MHz, CD₂Cl₂, -85 °C): δ = 16.94 (s, 1H, *H*(THF)₂), 9.22 (s, 1H, O*H*), 3.84 (br, 8H, OC*H*₂CH₂), 1.89 (br, 8H, OCH₂C*H*₂).
¹³C{¹H} NMR (75 MHz, CD₂Cl₂, -85 °C): δ = 152.6 (s, Ar-*C*), 150.5 (s, Ar-*C*), 144.9 (s, Ar-*C*), 141.3 (s, Ar-*C*), 124.9 (s, Ar-*C*), 124.4 (d, ²*J*_{TaC}= 13.2 Hz, Ar-*C*), 121.2 (s, Ar-*C*), 117.7 (s, Ar-*C*), 115.4 (s, Ar-*C*), 68.5 (s, O*C*H₂CH₂), 25.2 (s, OCH₂*C*H₂) ppm.

### Polymerization of monomers using initiator (V)

Polymerization of monomers with initiator (V) was performed by following the general procedure described above for initiator (III). Table 9 shows data for the polymerization of *n*-butyl vinyl ether, styrene and α-methyl styrene using initiator (V). Table 9 shows that good balance of high yield and high molecular weight for poly(n-butyl vinyl ether), poly(styrene) and poly(a-methylstyrene) can be achieved at temperatures much higher than -90°C.

**Table 9**

| Ex. | Monomer | T (°C) | [M]:[I] | Yield (%) | Mₙ (g/mol) | PDI |
|---|---|---|---|---|---|---|
| 45 | *n*-butyl vinyl ether | 20 | 400 | 13 | 20,400 | 1.59 |
| 46 | *n*-butyl vinyl ether | 0 | 400 | 48 | 19,300 | 1.61 |
| 47 | *n*-butyl vinyl ether | -50 | 400 | 66 | 28,100 | 2.07 |
| 48 | *n*-butyl vinyl ether | -78 | 400 | 62 | 117,000 | 1.13 |
| 49 | styrene | 19.8 | 400 | 83 | 14,400 | 1.86 |
| 50 | styrene | 0 | 400 | 83 | 26,600 | 1.69 |
| 51 | styrene | -50 | 400 | 6 | 143,600 | 1.31 |
| 52 | styrene | -78 | 400 | - | - | - |
| 53 | α-metyl styrene | 19 | 400 | 5 | 1,500 | 1.76 |
| 54 | α-metyl styrene | -78 | 400 | 23 | 84,900 | 1.45 |

### Initiator (VI):

### Synthesis of H((CH₃)₃COCH₃)₂[Ta(1,2-O₂C₆Cl₄)₂(1,2-O₂H₁C₆Cl₄)₂] (VI)

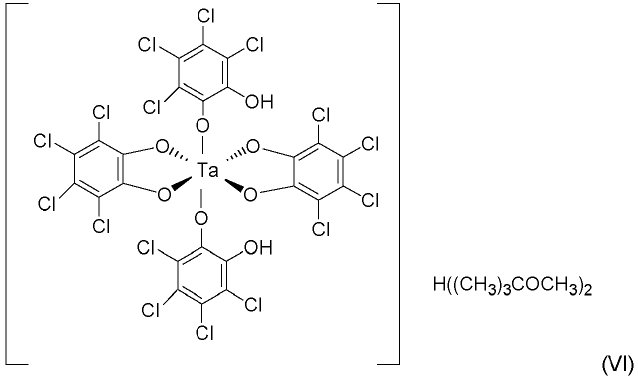

The synthesis of initiator (III) described above may be adapted to replace diethyl ether with methyl *tert*-butyl ether as the coordinating ligand for the proton to afford H((CH₃)₃COCH₃)₂[Ta(1,2-O₂C₆Cl₄)₂(1,2-O₂H₁C₆Cl₄)₂] (VI).

Thus, TaCl₅ (0.12 g, 0.35 mmol) was stirred in anhydrous CH₂Cl₂ (4 mL) and the white suspension was slowly heated to reflux under N₂ atmosphere. In another Schlenk flask, tetrachlorocatechol (0.33 g, 1.35 mmol) was prepared in warm anhydrous CH₂Cl₂ (6 mL) and the bright orange-red solution was added via cannula to the refluxing TaCl₅ solution at 90°C to afford a dark green reaction mixture. After 10 min, a colorless precipitate was obtained. The reaction mixture was refluxed for 120 min and cooled to ambient temperature. Upon addition of methyl *tert*-butyl ether (22 mL), a green clear solution formed. The solution was cooled in an ice bath to afford a small amount of a light green precipitate within 30 min. The reaction mixture was pumped down to dryness and washed with CH₂Cl₂ (1.5 mL) and dried in *vacuo.* Yield = (0.32 g, 0.24 mmol, 69 % based on TaCl₅).
¹H NMR (400 MHz, CD₂Cl₂, 25 °C): δ = 8.57 (br, O*H*), 3.20 (br, 6H, (C*H*₃)₃COCH₃)), 1.19 ppm (br, 18H, (CH₃)₃COC*H*₃)).
¹H NMR (400 MHz, CD₂Cl₂, -85 °C): δ = 18.11 (s, 1H, *H*[(CH₃)₃COCH₃)]₂), 9.94 (s, 1H, OH), 3.13(br, 6H, (C*H*₃)₃COCH₃)), 1.10 (br, 18H, (CH₃)₃COC*H*₃)) ppm.

### Polymerization of monomers using initiator (VI)

Polymerization of monomers with initiator (VI) was performed by following the general procedure described above for initiator (III). Table 10 shows data for the polymerization of n-butyl vinyl ether using initiator (VI). Table 10 shows that good balance of yield and high molecular weight for poly(n-butyl vinyl ether) can be achieved at temperatures higher than -90°C.

**Table 10**

| Ex. | Monomer | T (°C) | [M]:[I] | Yield (%) | Mₙ (g/mol) | PDI |
|---|---|---|---|---|---|---|
| 55 | *n*-butyl vinyl ether | -78 | 400 | 25 | 106,100 | 1.17 |

### Initiator (VII):

### Synthesis of H(Et₂O)₂[Nb(1,2-O₂C₆Cl₄)₂(1,2-O₂H₁C₆Cl₄)₂] (VII)

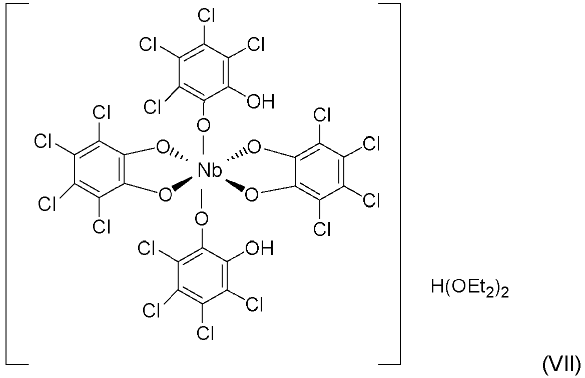

The synthesis of the initiator (III) described above may be adapted to replace the metal ion with niobium (Nb) to afford H(Et₂O)₂[Nb(1,2-O₂C₆Cl₄)₂(1,2-O₂H₁C₆Cl₄)₂] **(VII).**

Thus, NbCl₅ (0.13 g, 0.49 mmol) was stirred in anhydrous CH₂Cl₂ (6 mL) and the yellow suspension was slowly heated to reflux under N₂ atmosphere. In another Schlenk flask, tetrachlorocatechol (0.53 g, 2.14 mmol) was prepared in warm anhydrous CH₂Cl₂ (6 mL) and the bright orange-red solution was added via cannula to the refluxing NbCl₅ solution at 90°C to afford a dark red reaction mixture. The reaction mixture was refluxed for 100 min and cooled to ambient temperature. Et₂O (20 mL) was added and after stirring for 30 min, the solvent was removed under a reduced pressure at 0°C. The solid was collected by filtration, washed with CH₂Cl₂ (2 mL) and dried in *vacuo.* Yield = (0.25 g, 0.20 mmol, 40%).
¹H NMR (400 MHz, CD₂Cl₂, 25 °C): δ = 5.88 (br, O*H*), 3.56 (br, 8H, C*H*₂CH₃), 1.24 ppm (br, 12H, CH₂C*H*₃).
¹H NMR (400 MHz, CD₂Cl₂, -85 °C): δ = 16.75 (s, 1H, *H*(Et₂O)₂), 9. (s, 1H, O*H*), 4.13 (br, 8H, C*H*₂CH₃), 1.44 (br, 12H, CH₂C*H*₃) ppm.

### Polymerization of monomers using initiator (VII)

Polymerization of monomers with initiator (VII) was performed by following the general procedure described above for initiator (III). Table 11 shows data for the polymerization of *n*-butyl vinyl ether and styrene using initiator (VII). Table 11 shows that the niobium complex can also initiate cationic polymerization of *n*-butyl vinyl ether and styrene.

**Table 11**

| Ex. | Monomer | T (°C) | [M]:[I] | Yield (%) | Mₙ (g/mol) | PDI |
|---|---|---|---|---|---|---|
| 56 | *n*-butyl vinyl ether | -78 | 400 | 71 | 41,900 | 1.09 |
| 57 | styrene | -78 | 400 | 6.4 | 112,500 | 1.42 |

### Initiator (VIII):

### Synthesis of H(OEt₂)₂[Ta(1,2-O₂C₆H₃F)₂(1,2-O₂C₆H₄F)₂] (VIII)

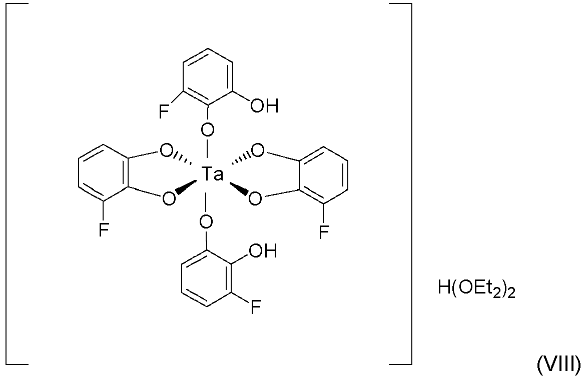

The reaction of 4 equivalents of monofluorocatechol with TaCl₅ in a manner as described for the synthesis of the analog (III) affords the corresponding H(OEt₂)₂[Ta(1,2-O₂C₆H₃F)₂(1,2-O₂C₆H₄F)₂] (VIII).

Thus, TaCl₅ (0.25 g, 0.69 mmol) was stirred in anhydrous CH₂Cl₂ (5 mL) and the white suspension was slowly heated to reflux under N₂ atmosphere. In another Schlenk flask, 3-fluorocatechol (0.34 g, 2.67 mmol) was prepared in warm anhydrous CH₂Cl₂ (5 mL) and the colourless solution was added via cannula to the refluxing TaCl₅ solution at 90°C to afford an orange solution. After 10 min, a colorless precipitate was obtained. The reaction mixture was refluxed for 80 min and cooled to ambient temperature. Upon addition of Et₂O (15 mL), a yellow clear solution formed. The solution was cooled in an ice bath to afford an off-white precipitate within 15 min. The solid was collected by filtration, washed with CH₂Cl₂ (2 mL) and dried in *vacuo.* Yield = (0.12 g, 0.14 mmol, 20 %).
¹H NMR (400 MHz, CD₂Cl₂, -80 °C): δ = 18.75 (s, 1H, *H*(Et₂O)₂), 10.41 (s, 1H, OH), 6.00-7.00 (m, Ar-H), 4.22 (br, 8H, C*H*₂CH₃), 1.55 (br, 12H, CH₂C*H*₃) ppm.

### Initiator (IX):

### Synthesis of H(OEt₂)₂[Ta(1,2-O₂C₆H₄)₂(1,2-O₂C₆H₅)₂]/H[(OEt₂)]₂[Ta(1,2-O₂C₆H₄)₃] (IX)

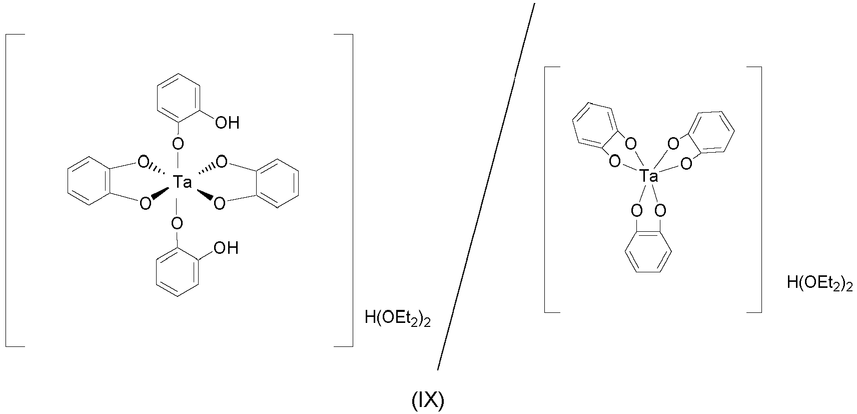

The reaction of 4 equivalents of catechol with TaCl₅ in a manner as described for the synthesis of the chlorinated analog (III) affords a mixture (IX) of the corresponding non-halogenated H(OEt₂)₂[Ta(1,2-O₂C₆H₄)₂(1,2-O₂C₆H₅)₂] and a tantalum complex coordinated with three bidentate catechol ligands but no monodentate catechol ligands.

Thus, TaCl₅ (0.81 g, 22.7 mmol) was stirred in anhydrous CH₂Cl₂ (6 mL) and the white suspension was slowly heated to reflux under N₂ atmosphere. In another Schlenk flask, catechol (1.00 g, 90.8 mmol) was prepared in a solvent mixture containing anhydrous CH₂Cl₂ (6 mL) and anhydrous toluene (8 mL) and the bright orange-red solution mixture was warmed up to 50°C and added via cannula to the refluxing TaCl₅ solution at 90°C to afford a dark orange reaction mixture. After 10 min, a colorless precipitate was obtained. The reaction mixture was refluxed for 60 min and cooled to ambient temperature. The reaction mixture was stirred for another 120 min at ambient temperature. Upon addition of diethyl ether (18 mL), a yellow clear solution formed. The solution was cooled in an ice bath to afford a yellow precipitate within 30 min. The solid was collected by filtration, washed with CH₂Cl₂ (2 mL) and dried in *vacuo.* Yield = (0.58 g).
¹H NMR (400 MHz, CD₂Cl₂, 25 °C): δ = 8.07-6.28 (m, Ar-*H*), 3.62 (br, 8H, OC*H*₂CH₃), 1.24 (t, ³*J*_{HH} = 6.7H, OCH₂C*H*₃). ¹H NMR (400 MHz, CD₂Cl₂, -85 °C): δ = 15.57 (s, 1H, *H*(OEt₂)₂), 10.28 (s, OH), 8.27-6.78 (m, Ar-*H*), 4.19 (br, 8H, OC*H*₂CH₃), 1.51 ppm (br, 12H, OCH₂C*H*₃) ppm.

### Polymerization of monomers using a mixture of initiator (IX) with the corresponding 3-ligand tantalum complex

Polymerization of monomers with initiator (IX) was performed by following the general procedure described above for initiator (III). Table 12 shows data for the polymerization of *n*-butyl vinyl ether, styrene and α-methyl styrene using initiator (IX) together with the corresponding 4-ligand tantalum complex. Table 12 shows that the balance of yield and molecular weight are generally poorer than for the chlorinated analog (III).

**Table 12**

| Ex. | Monomer | T (°C) | [M]:[I] | Yield (%) | Mₙ (g/mol) | PDI |
|---|---|---|---|---|---|---|
| 58 | *n*-butyl vinyl ether | 19.6 | 400 | 29 | 14,500 | 1.48 |
| 59 | *n*-butyl vinyl ether | -78 | 400 | 17 | 96,400 | 1.33 |
| 60 | styrene | 19.6 | 400 | <1 | 43,300 | 1.28 |
| 61 | styrene | -50 | 400 | 1.2 | n.d. | n.d. |
| 62 | α-metyl styrene | 19.6 | 400 | <1 | 6,000 | 4.18 |
| 63 | α-metyl styrene | -50 | 400 | 1.7 | 10,900 | 1.60 |
| 64 | α-methyl styrene | -78 | 400 | 1.8 | 31,500 | 1.32 |

| | | | | | | |
|---|---|---|---|---|---|---|
| n.d. = not determined | | | | | | |

### Use of Initiators (III), (IV) and (VII) to Polymerize Isobutylene:

Isobutylene polymers (PIB) and isobutylene-isoprene copolymers (IIR - butyl rubber) were prepared using Initiators (III), (IV) and (VII) by the following procedure.

Initiator (100 mg) was stirred in anhydrous CH₂Cl₂ (25 mL) for 30 minutes at -30°C. In another reaction flask, 6 mL of dry isobutylene (or 6 mL of dry isobutylene and 0.25 mL of isoprene when producing IIR) and 50 mL CH₂Cl₂ was stirred at -30°C, then 7 mL of the initiator solution was added. The reaction mixture was stirred for 17 minutes at - 30°C. Afterwards, the polymerization was stopped by adding 1.0 mL ethanol containing 1 Molar tetrakis-[methylene-(3,5-di-*tert*-butyl-4-hydroxyhydrocinnamate)] methane (CAS# 6683-19-8). The solvent was evaporated from the reaction mixture. The polymer residue was dissolved in hexane, filtered, and then the hexane removed to provide a polymer. Table 13 shows data for the preparation of PIB and IIR.

**Table 13**

| Ex. | Monomer | Initiator | Yield (%) | Mₙ (g/mol) | PDI |
|---|---|---|---|---|---|
| 65 | isobutylene | (III) | 62 | 1,200 | 2.96 |
| 66 | isobutylene | (IV) | 8 | 3,400 | 2.18 |
| 67 | isobutylene | (VII) | 20 | 4,000 | 1.98 |

With reference to Fig. 1, the ¹H NMR spectrum of the polyisobutylene (PIB) produced in Ex. 67 is reactive PIB, having no terminal chloride. The PIB has a considerable proportion of terminal ethylenic unsaturation. The initiators therefore provide the opportunity to produce PIB and butyl polymers with reactive ends.

## Claims

1. A Brønsted-Lowry acid initiator system for cationic polymerization of an ethylenically unsaturated monomer, the Brønsted-Lowry acid initiator system comprising an initiator having a structure of Formula (I) in an anhydrous polymerization medium: 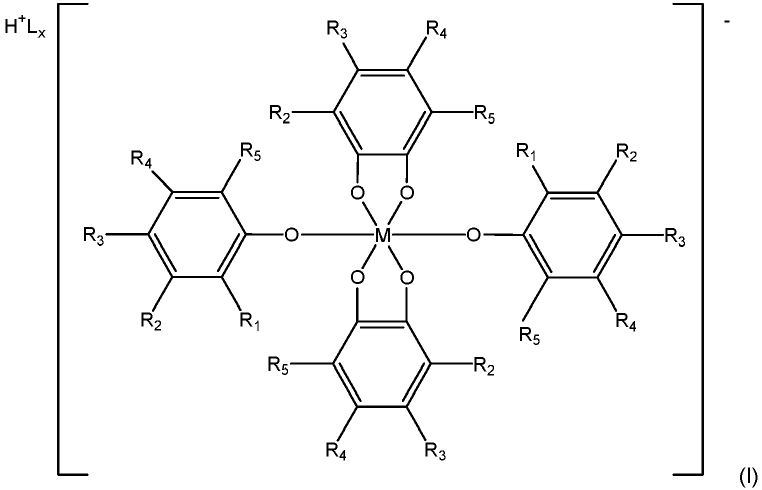 where:
M is tantalum (Ta), vanadium (V) or niobium (Nb);
each R₁ is independently H, OR₆, F, Cl, Br, I or alkyl, where R₆ is H or alkyl;
R₂, R₃, R₄ and R₅ are the same or different and are independently selected from H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present
wherein alkyl is selected from C₁-C₆ alkyls which may be non-substituted or substituted by one or more substituents selected from F, Cl, Br and aryl,
and wherein the aryl is selected from phenyl and phenyls that are substituted by one or more substituent selected from F, Cl, Br and C₁-C₆ alkyls, which may be non-substituted or substituted by one or more substituents selected from F, Cl, and Br.

2. The system according to claim 1, wherein M is Ta.

3. The system according to claim 1 or 2, wherein L is a stabilizing molecule for the H⁺ having one or more lone pairs of electrons; preferably L is a sterically-hindered molecule.

4. The system according to claim 1 or 2, wherein L is an alkyl ether or a cycloalkyl ether.

5. The system according to claim 1 or 2, wherein L is diethyl ether.

6. The system according to any one of claims 1 to 5, wherein R₂, R₃, R₄ and R₅ are the same and are F or CI.

7. The system according to claim 1, wherein: M is Ta; both of R₁ are OH; R₂, R₃, R₄ and R₅ are Cl; L is Et₂O; and, x is 2.

8. The system according to any one of claims 1 to 7, wherein the polymerization medium comprises dichloromethane or methyl chloride; and/or the system contains substantially no water.

9. A process for producing a polymer, the process comprising polymerizing one or more ethylenically unsaturated monomers with the initiator system as defined in any one of claims 1 to 8; preferably the polymerization is performed at a temperature of -85 °C or higher.

10. A compound of Formula (I): 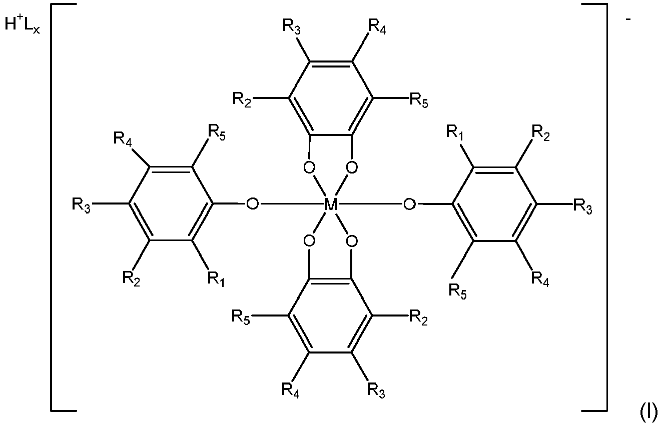 where:
M is tantalum (Ta), vanadium (V) or niobium (Nb);
each R₁ is independently H, OR₆, F, Cl, Br, I or alkyl, where R₆ is H or alkyl;
R₂, R₃, R₄ and R₅ are the same or different and are independently selected from H, F, Cl, Br, I, alkyl or aryl, or two or more of R₂, R₃, R₄ and R₅ on a same benzene ring are taken together to form a bicyclic, tricyclic or tetracyclic moiety with the benzene ring, with the proviso that all of R₂, R₃, R₄ and R₅ on the same benzene ring are not H;
L is absent or a molecule that coordinates to H⁺; and,
x is 0 when L is absent, or x is 0.5 or more when L is present,
wherein alkyl is selected from C₁-C₆ alkyls which may be non-substituted or substituted by one or more substituents selected from F, Cl, Br and aryl,
and wherein the aryl is selected from phenyl and phenyls that are substituted by one or
more substituent selected from F, Cl, Br and C₁-C₆ alkyls, which may be non-substituted or substituted by one or more substituents selected from F, Cl, and Br.

11. The compound according to claim 10, wherein M is Ta.

12. The compound according to claim 10 or 11, wherein L is a stabilizing molecule for the H⁺ having one or more lone pairs of electrons; preferably L is a sterically-hindered molecule.

13. The compound according to claim 10 or 11, wherein L is an alkyl ether or a cycloalkyl ether.

14. The compound according to any one of claims 10 to 13, wherein R₂, R₃, R₄ and R₅ are the same and are F or Cl.

15. The compound according to claim 10, wherein: M is Ta; both of R₁ are OH; R₂, R₃, R₄ and R₅ are Cl; L is Et₂O; and, x is 2.

## Patentansprüche

1. Brönsted-Lowry-Säure-Initiatorsystem für die kationische Polymerisation eines ethylenisch ungesättigten Monomers, wobei das Brönsted-Lowry-Säure-Initiatorsystem einen Initiator mit einer Struktur der Formel (I) in einem wasserfreien Polymerisationsmedium umfasst: 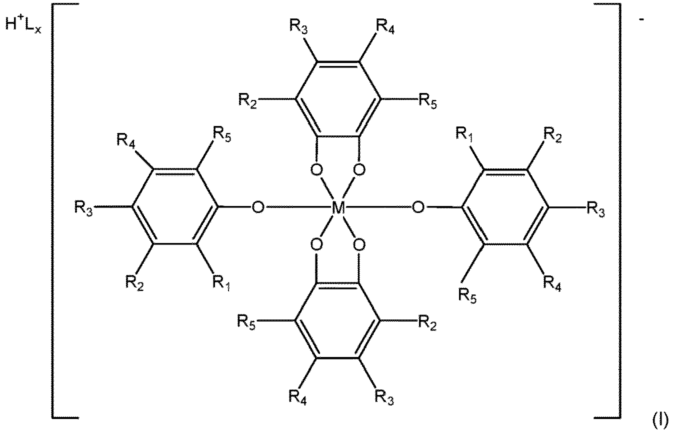 wobei:
M für Tantal (Ta), Vanadium (V) oder Niob (Mb) steht;
R₁ jeweils unabhängig für H, OR₆, F, Cl, Br, I oder Alkyl steht, wobei R₆ für H oder Alkyl steht;
R₂, R₃, R₄ und R₅ gleich oder verschieden sind und unabhängig aus H, F, Cl, Br, I, Alkyl oder Aryl ausgewählt sind oder zwei oder mehr von R₂, R₃, R₄ und R₅ an demselben Benzolring zusammengenommen mit dem Benzolring eine bicyclische, tricyclische oder tetracyclische Gruppierung bilden, mit der Maßgabe, dass alle von R₂, R₃, R₄ und R₅ nicht für H stehen;
L fehlt oder für ein an H⁺ koordinierendes Moleküls steht; und
x für 0 steht, wenn L fehlt, oder x für 0,5 oder mehr steht, wenn L vorhanden ist;
wobei das Alkyl aus C₁-C₆-Alkylgruppen, die unsubstituiert oder durch einen oder mehrere Substituenten, die aus F, Cl, Br und Aryl ausgewählt sind, substituiert sein können, ausgewählt ist, und wobei das Aryl aus Phenyl und substituierten Phenylen ausgewählt ist, die durch einen oder mehrere Substituenten, die aus F, Cl, Br und C₁-C₆-Alkylgruppen ausgewählt sind, substituiert sind, wobei die C₁-C₆-Alkylgruppen unsubstituiert oder durch einen oder mehrere Substituenten, die aus F, Cl, Br und Aryl ausgewählt sind, substituiert sein können.

2. System nach Anspruch 1, wobei M für Ta steht.

3. System nach Anspruch 1 oder 2, wobei L für ein stabilisierendes Molekül für das H⁺ mit einem oder mehreren einsamen Elektronenpaaren steht; vorzugsweise L für ein sterisch gehindertes Molekül steht.

4. System nach Anspruch 1 oder 2, wobei L für einen Alkylether oder einen Cycloalkylether steht.

5. System nach Anspruch 1 oder 2, wobei L für Diethylether steht.

6. System nach einem der Ansprüche 1 bis 5, wobei R₂, R₃, R₄ und R₅ gleich sind und für F oder Cl stehen.

7. System nach Anspruch 1, wobei: M für Ta steht; beide R₁ für OH stehen; R₂, R₃, R₄ und R₅ für Cl stehen; L für Et₂O steht und x für 2 steht.

8. System nach einem der Ansprüche 1 bis 7, wobei das Polymerisationsmedium Dichlormethan oder Methylchlorid umfasst und/oder das System praktisch kein Wasser enthält.

9. Verfahren zur Herstellung eines Polymers, wobei das Verfahren das Polymerisieren eines oder mehrerer ethylenisch ungesättigter Monomere mit einem Initiatorsystem gemäß einem der Ansprüche 1 bis 8 umfasst; vorzugsweise die Polymerisation einer Temperatur von -85 °C oder darüber durchgeführt wird.

10. Verbindung der Formel (I): 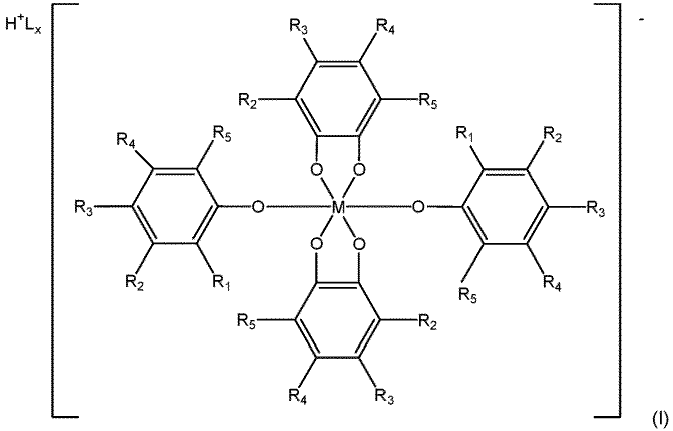 wobei:
M für Tantal (Ta), Vanadium (V) oder Niob (Mb) steht;
R₁ jeweils unabhängig für H, OR₆, F, Cl, Br, I oder Alkyl steht, wobei R₆ für H oder Alkyl steht;
R₂, R₃, R₄ und R₅ gleich oder verschieden sind und unabhängig aus H, F, Cl, Br, I, Alkyl oder Aryl ausgewählt sind oder zwei oder mehr von R₂, R₃, R₄ und R₅ an demselben Benzolring zusammengenommen mit dem Benzolring eine bicyclische, tricyclische oder tetracyclische Gruppierung bilden, mit der Maßgabe, dass alle von R₂, R₃, R₄ und R₅ nicht für H stehen;
L fehlt oder für ein an H⁺ koordinierendes Moleküls steht; und
x für 0 steht, wenn L fehlt, oder x für 0,5 oder mehr steht, wenn L vorhanden ist;
wobei Alkyl aus C₁-C₆-Alkylgruppen, die unsubstituiert oder durch einen oder mehrere Substituenten, die aus F, Cl, Br und Aryl ausgewählt sind, substituiert sein können, ausgewählt ist, und wobei das Aryl aus Phenyl und substituierten Phenylen ausgewählt ist, die durch einen oder mehrere Substituenten, die aus F, Cl, Br und C₁-C₆-Alkylgruppen ausgewählt sind, substituiert sind, wobei die C₁-C₆-Alkylgruppen unsubstituiert oder durch einen oder mehrere Substituenten, die aus F, Cl, Br und Aryl ausgewählt sind, substituiert sein können.

11. Verbindung nach Anspruch 10, wobei M für Ta steht.

12. Verbindung nach Anspruch 10 oder 11, wobei L für ein stabilisierendes Molekül für das H⁺ mit einem oder mehreren einsamen Elektronenpaaren steht; vorzugsweise L für ein sterisch gehindertes Molekül steht.

13. Verbindung nach Anspruch 10 oder 11, wobei L für einen Alkylether oder einen Cycloalkylether steht.

14. Verbindung nach einem der Ansprüche 10 bis 13, wobei R₂, R₃, R₄ und R₅ gleich sind und für F oder Cl stehen.

15. Verbindung nach Anspruch 10, wobei: M für Ta steht; beide R₁ für OH stehen; R₂, R₃, R₄ und R₅ für Cl stehen; L für Et₂O steht und x für 2 steht.

## Revendications

1. Système initiateur acide de Brønsted-Lowry pour la polymérisation cationique d'un monomère à insaturation éthylénique, le système initiateur acide de Brønsted-Lowry comprenant un initiateur ayant une structure de formule (I) dans un milieu de polymérisation anhydre : 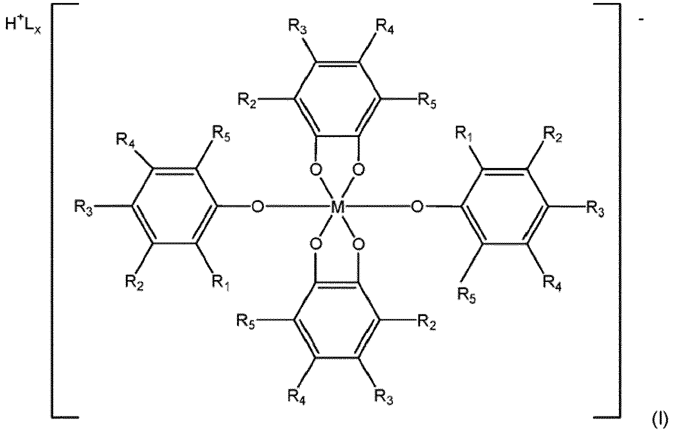 où :
M est le tantale (Ta), le vanadium (V) ou le niobium (Nb) ;
chaque R₁ est indépendamment H, OR₆, F, Cl, Br, I ou un groupe alkyle, où R₆ est H ou un groupe alkyle ;
R₂, R₃, R₄ et R₅ sont identiques ou différents et sont indépendamment choisis parmi H, F, Cl, Br, I, un groupe alkyle ou un groupe aryle ou deux ou plus de deux de R₂, R₃, R₄ et R₅ sur un même noyau benzénique sont pris ensemble pour former une fraction bicyclique, tricyclique ou tétracyclique avec le noyau benzénique, à condition que R₂, R₃, R₄ et R₅ sur le même noyau benzénique ne soient pas tous H ;
L est absent ou est une molécule qui forme une liaison de coordination avec H⁺ ; et
x est 0 lorsque L est absent ou x est supérieur ou égal à 0,5 lorsque L est présent,
dans lequel le groupe alkyle est choisi parmi les groupes alkyle en C₁-C₆ qui peuvent être non substitués ou substitués par un ou plusieurs substituants choisis parmi F, Cl, Br et un groupe aryle et
dans lequel le groupe aryle est choisi parmi le groupe phényle et les groupes phényle qui sont substitués par un ou plusieurs substituants choisis parmi F, Cl, Br et les groupes alkyle en C₁-C₆, qui peuvent être non substitués ou substitués par un ou plusieurs substituants choisis parmi F, Cl et Br.

2. Système selon la revendication 1, dans lequel M est Ta.

3. Système selon la revendication 1 ou 2, dans lequel L est une molécule de stabilisation pour le H⁺ ayant une ou plusieurs paires d'électrons célibataires ; de préférence L est une molécule stériquement encombrée.

4. Système selon la revendication 1 ou 2, dans lequel L est un éther d'alkyle ou un éther de cycloalkyle.

5. Système selon la revendication 1 ou 2, dans lequel L est l'éther de diéthyle.

6. Système selon l'une quelconque des revendications 1 à 5, dans lequel R₂, R₃, R₄ et R₅ sont identiques et sont F ou Cl.

7. Système selon la revendication 1, dans lequel : M est Ta ; les deux R₁ sont OH ; R₂, R₃, R₄ et R₅ sont Cl ; L est Et₂O ; et x est 2.

8. Système selon l'une quelconque des revendications 1 à 7, dans lequel le milieu de polymérisation comprend du dichlorométhane ou du chlorure de méthyle ; et/ou le système ne contient pratiquement pas d'eau.

9. Procédé pour la production d'un polymère, le procédé comprenant la polymérisation d'un ou plusieurs monomères à insaturation éthylénique avec le système initiateur tel que défini dans l'une quelconque des revendications 1 à 8 ; de préférence la polymérisation étant effectuée à une température supérieure ou égale à -85 °C.

10. Composé de formule (I) : 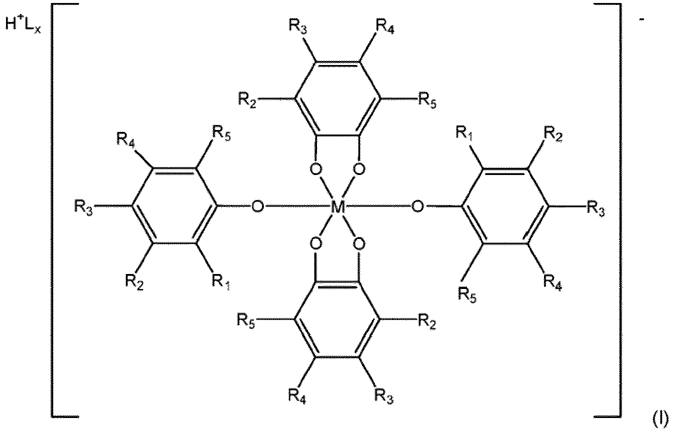 où :
M est le tantale (Ta), le vanadium (V) ou le niobium (Nb) ;
chaque R₁ est indépendamment H, OR₆, F, Cl, Br, I ou un groupe alkyle, où R₆ est H ou un groupe alkyle ;
R₂, R₃, R₄ et R₅ sont identiques ou différents et sont indépendamment choisis parmi H, F, Cl, Br, I, un groupe alkyle ou un groupe aryle ou deux ou plus de deux de R₂, R₃, R₄ et R₅ sur un même noyau benzénique sont pris ensemble pour former une fraction bicyclique, tricyclique ou tétracyclique avec le noyau benzénique, à condition que R₂, R₃, R₄ et R₅ sur le même noyau benzénique ne soient pas tous H ;
L est absent ou est une molécule qui forme une liaison de coordination avec H⁺ ; et
x est 0 lorsque L est absent ou x est supérieur ou égal à 0,5 lorsque L est présent,
dans lequel le groupe alkyle est choisi parmi les groupes alkyle en C₁-C₆ qui peuvent être non substitués ou substitués par un ou plusieurs substituants choisis parmi F, Cl, Br et un groupe aryle et
dans lequel le groupe aryle est choisi parmi le groupe phényle et les groupes phényle qui sont substitués par un ou plusieurs substituants choisis parmi F, Cl, Br et les groupes alkyle en C₁-C₆, qui peuvent être non substitués ou substitués par un ou plusieurs substituants choisis parmi F, Cl et Br.

11. Composé selon la revendication 10, dans lequel M est Ta.

12. Composé selon la revendication 10 ou 11, dans lequel L est une molécule de stabilisation pour le H⁺ ayant une ou plusieurs paires d'électrons célibataires ; de préférence L est une molécule stériquement encombrée.

13. Composé selon la revendication 10 ou 11, dans lequel L est un éther d'alkyle ou un éther de cycloalkyle.

14. Composé selon l'une quelconque des revendications 10 à 13, dans lequel R₂, R₃, R₄ et R₅ sont identiques et sont F ou Cl.

15. Composé selon la revendication 10, dans lequel : M est Ta ; les deux R₁ sont OH ; R₂, R₃, R₄ et R₅ sont Cl ; L est Et₂O ; et x est 2.
